# EUROPEAN PATENT SPECIFICATION

(11) **EP 3 094 621 B1**
(45) Date of publication and mention of the grant of the patent: **19.06.2019**
(21) Application number: 15708055.7
(22) Date of filing: 13.01.2015
(51) Int. Cl.: C07D 213/74, C07D 239/54, C07D 409/12, G01N 21/3581, G01N 21/3586, G01N 21/359

(54) **USE OF N-(PYRIDIN-2-YL)-AMIDE DERIVATIVES FOR TERAHERTZ TAGGING APPLICATIONS**
VERWENDUNG VON N-(PYRIDIN-2-YL)-AMID-DERIVATEN FÜR TERAHERZ-MARKIERUNGS-ANWENDUNGEN
UTILISATION DE DÉRIVÉS DE N-(PYRIDIN-2-YL)-AMIDE POUR APPLICATIONS DE MARQUAGE PAR TÉRAHERTZ

(30) Priority: 13.01.2014 IN 95DE2014
(43) Date of publication of application: 23.11.2016
(60) Divisional application: 18169326.8
(73) Proprietor: Council of Scientific & Industrial Research, New Delhi 110001 (IN)
(72) Inventor: AMBADE, Ashootosh Vasant, Pune Maharashtra 411008 (IN); PESALA, Bala, Chennai 600113 (IN); JOSHI, Kavita, Pune Maharashtra 411008 (IN); BASUTKAR, Nitin Bapurao, Pune Maharashtra 411008 (IN); RAY, Shaumik, Chennai 600113 (IN); DASH, Jyotirmayee, Chennai 600113 (IN); VILASRAO, Kaware Vaibhav, Pune Maharashtra 411008 (IN)
(74) Representative: Høiberg P/S
(86) International application number: PCT/IN2015/050005
(87) International publication number: WO 2015/104722

(56) References cited:
- WO-A1-2004/081545
- WO-A1-2013/106728
- WO-A2-2010/020407
- WO-A2-2011/004393
- US-A- 3 776 917
- US-A- 3 979 408
- US-A- 4 132 713
- US-A1- 2008 039 633
- LANGER, PETER ET AL: "3,5,7,9-Tetraphenylhexaazaacridine: A highly stable, weakly antiaromatic species with 16 .pi. electrons", ANGEWANDTE CHEMIE, INTERNATIONAL EDITION, vol. 44, no. 33, 2005, pages 5255-5259, XP002738220, ISSN: 1433-7851, DOI: 10.1002/ANIE.200500831
- BORYS OSMIALOWSKI ET AL: "Complexation of 2,6-Bis(acylamino)pyridines with Dipyridin-2-ylamine and 4,4-Dimethylpiperidine-2,6-dione", THE JOURNAL OF PHYSICAL CHEMISTRY A, vol. 114, no. 49, 16 December 2010 (2010-12-16), pages 12881-12887, XP055181667, ISSN: 1089-5639, DOI: 10.1021/jp1084857
- CHEN, DONGZHONG ET AL: "Supramolecular liquid crystals with hydrogen-bonding self-assembled T-shaped mesogens", CHEMISTRY LETTERS, vol. 11, 2001, pages 1156-1157, XP002738221, ISSN: 0366-7022, DOI: 10.1246/CL.2001.1156
- PANAGIOTIS MANESIOTIS ET AL: "Improved Imide Receptors by Imprinting Using Pyrimidine-Based Fluorescent Reporter Monomers", THE JOURNAL OF ORGANIC CHEMISTRY, vol. 70, no. 7, 1 April 2005 (2005-04-01), pages 2729-2738, XP055181778, ISSN: 0022-3263, DOI: 10.1021/jo0477906
- YANG SIZHUO ET AL: "Copper-catalyzed dehydrogenative reaction: synthesis of amide from aldehydes and aminopyridine", TETRAHEDRON, vol. 69, no. 31, 23 May 2013 (2013-05-23), pages 6431-6435, XP028570785, ELSEVIER SCIENCE PUBLISHERS, AMSTERDAM, NL ISSN: 0040-4020, DOI: 10.1016/J.TET.2013.05.072
- N. LAMAN ET AL: "High-Resolution Waveguide THz Spectroscopy of Biological Molecules", BIOPHYSICAL JOURNAL, vol. 94, no. 3, 1 February 2008 (2008-02-01), pages 1010-1020, XP055181841, ISSN: 0006-3495, DOI: 10.1529/biophysj.107.113647

## Description

### FIELD

The present disclosure relates to compounds selected from a list of compounds (a) to (i), (aa), (cc) and binary molecular complexes based thereon with varying molecular mass and hydrogen bond strengths demonstrating several resonances below 10 THz which are customizable for various applications.

### BACKGROUND AND PRIOR ART

THz spectroscopy has been used to study a variety of physical phenomena ranging from atomic transitions to dynamics of biological molecules, and hence involves a wide range of disciplines including physics, chemistry, engineering, astronomy, biology, and medicine.

Terahertz (THz) frequency band lies between the microwave and mid-infrared region of the electromagnetic spectrum. Molecules having strong resonances in this frequency range are ideal for realizing Terahertz tags which can be easily incorporated into various materials. These THz tags find novel use in various counterfeiting applications such as detection of fake currency notes, security documents and counterfeit pharmaceutical drugs, brand protection, and labeling of consumer and industrial products/solutions. The absence of THz signatures for organic materials typically used in consumer/industrial labeling products also makes the present approach advantageous since the embedded THz tags can be easily detected.

THz spectroscopy of molecules, especially at frequencies below 10 THz provides valuable information on the low frequency vibrational modes, viz. intermolecular vibrational modes, intramolecular vibrational modes, hydrogen-bond stretching, torsional vibrations in several chemical and biological compounds. So far there have been very few attempts to engineer molecules which can demonstrate customizable resonances in the THz frequency region.

An article titled, "Application of terahertz spectroscopy and molecular modeling in isomers investigation: Glucose and fructose" by Z.-P. Zheng et al. in Optics Communications 285 (2012) 1868-1871 reports the THz spectra of glucose and fructose in the frequency region from 0.5 to 4.0 THz by THz-TDS at room temperature and employs the gaseous-state theory to simulate the isolated molecules of glucose and fructose.

Another article titled, "Discrimination of Chiral Solids: A Terahertz Spectroscopic Investigation of L- and DL-Serine" by King et al. in J. Phys. Chem. A, 2010, 114, 2945-2953 reports THz absorption spectra from 10 to 90 cm⁻¹ for L- and DL-serine along with a complete computational analysis by solid-state DFT using periodic boundary conditions.

Another article titled "N. Laman et al: "High-Resolution Waveguide THz Spectroscopy of Biological molecules", Biophysical Journal, vol. 94, no. 3, 1February2008 (2008-02-01), pages 1010-1020, XP055181841, ISSN: 0006-3495, DOI: 10.1529/ biophysj.107.113647 discloses tagging material which is known for terahertz resonance. Thymine itself is known to have a significant terahertz resonance.

WO 2004/081545 A1 (Univ Glasgow [GB]; Cumming David Robert Sime [GB]; Drysdale Timothy DA) 23 September 2004 (2004-09-23) and WO 2010/020407 A2 (Bendele Thomas [DE]; Bendele Tanja [DE]) 25 February 2010 (2010-02-25) describes the terahertz tagging for authentication of products.

Molecules having strong resonances in the THz frequency range are ideal for realizing Terahertz tags. These THz tags find novel use in various anti-counterfeiting applications such as detection of fake currency notes, security documents and counterfeit pharmaceutical drugs, brand protection, and labeling of consumer and industrial products/solutions.

However, there have been very few attempts to engineer molecules which can demonstrate customizable THz resonances. Hence, new molecules with unique and customizable spectroscopic signatures in the Terahertz region are required for incorporation as tags in various anti-counterfeiting applications (currency, pharmaceutical drugs, automotive parts, brand protection, and labeling of consumer and industrial products/solutions, etc.)

### OBJECTIVE

The main object of the present disclosure is to provide organic molecules with various molecular masses and exhibiting various functional groups and tunable strength of various intra/intermolecular bonds thereby resulting in highly customizable and specific signatures in the Terahertz region (<10 THz).

Another object of the present disclosure is to provide a simplified methodology to predict novel molecular structures that can result in strong signatures in the THz region.

### SUMMARY

Accordingly, present disclosure provides compounds selected from a list of compounds (a) to (i), (aa), (cc) and binary molecular complexes based on the said compounds with varying mass and intra/inter-molecular, hydrogen bond strengths demonstrating several resonances below 10 THz.

### BRIEF DESCRIPTION OF THE DRAWINGS

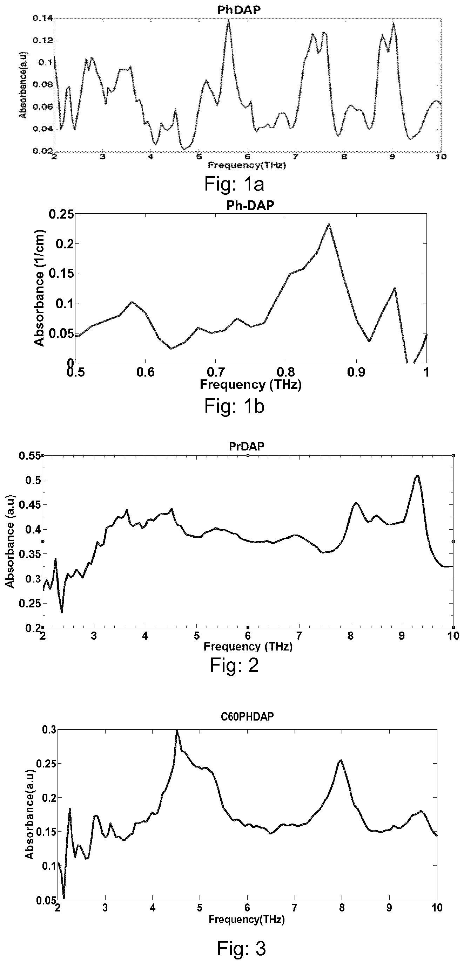

**Fig 1****:** THz spectrum of *N,N'*-(pyridine-2,6-diyl)dibenzamide (PhDAP) in the range 2-10 THz (a) and below 1THz (b).
**Fig 2****:** THz spectrum of *N,N'*-(pyridine-2,6 diyl)dipropionamide (PrDAP).
**Fig 3****:** THz spectrum of *N,N'*-(pyridine-2,6-diyl)bis(4-(hexyloxy)benzamide) (C6OPhDAP).

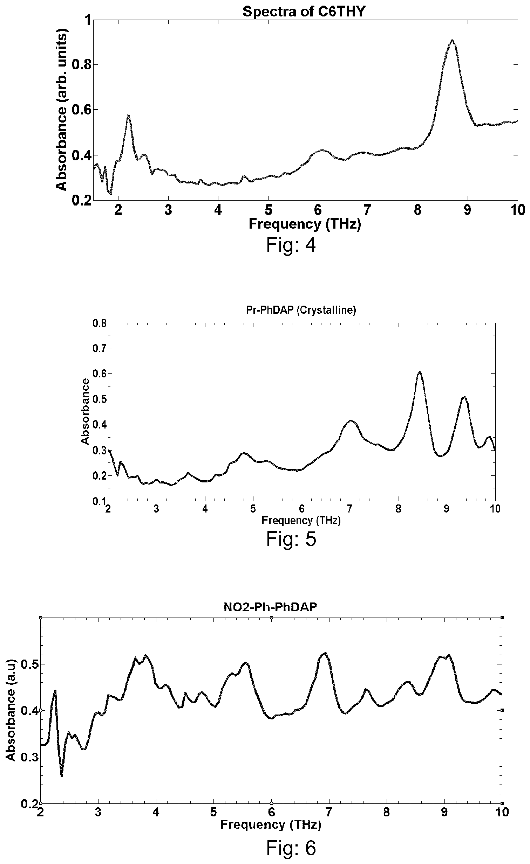

**Fig 4****:** THz spectrum of 1-hexyl-5-methylpyrimidine-2,4(1H, 3H)-dione (C6THY).
**Fig 5****:** THz spectrum of N-(6-propionamidopyridin-2-yl)benzamide (Pr-PhDAP).
**Fig 6****:** THz spectrum of N-(6-benzamidopyridin-2-yl)-4-nitrobenzamide (4-NO₂Ph-PhDAP).
**Fig 7****:** THz spectrum of N-(6-(3-phenylureido)pyridin-2-yl)benzamide (PhU-PhAP).
**Fig 8****:** THz spectrum of 4-fluoro-N-(6-propionamidopyridin-2-yl)benzamide (4-FPh-PrDAP)
**Fig 9****:** THz spectrum of 4-chloro-N-(6-propionamidopyridin-2-yl)benzamide (4-ClPh-PrDAP)
**Fig 10****:** THz spectrum of 4-bromo-N-(6-propionamidopyridin-2-yl)benzamide (4-BrPh-PrDAP)
**Fig 11****:** THz spectrum of 4-iodo-N-(6-propionamidopyridin-2-yl)benzamide (4-IPh-PrDAP)
**Fig 12****:** THz spectrum of 4-nitro-N-(6-propionamidopyridin-2-yl)benzamide (4-NO₂Ph-PrDAP)
**Fig 13****:** THz spectrum of N,N'-(pyridine-2,6-diyl)bis(thiophene-2-carboxamide) (Th-DAP)
**Fig 14****:** THz spectrum of PhDAP+C6Thy (1:1 complex).
**Fig 15****:** THz spectrum of C6OPhDAP+C6Thy (1:1 complex).

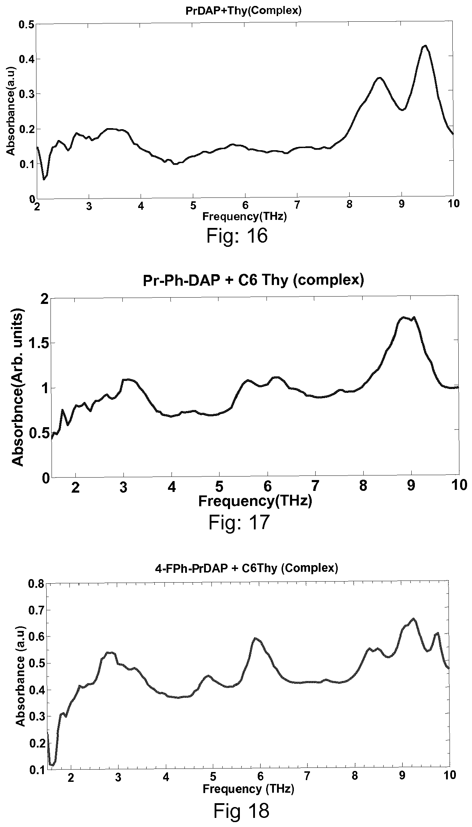

**Fig 16****:** THz spectrum of PrDAP+ C6Thy (1:1 complex).
**Fig 17****:** THz spectrum of Pr-PhDAP+C6Thy (1:1 complex).
**Fig 18****:** THz spectrum of 4-FPh-PrDAP+C6Thy (1:1 complex).

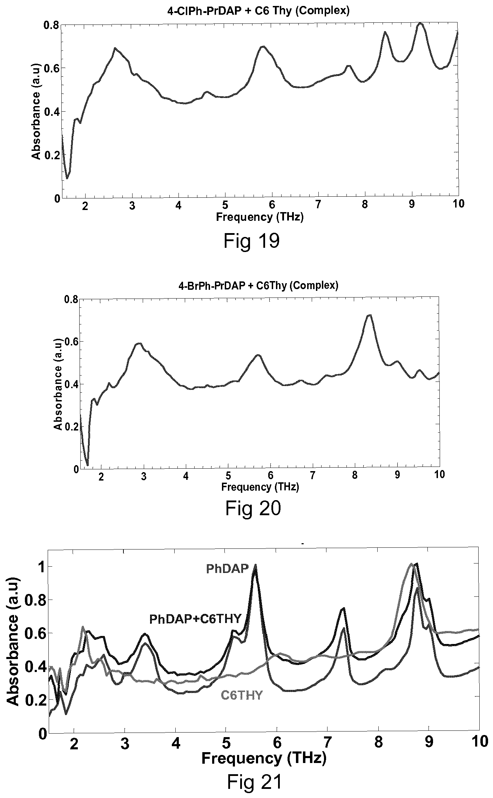

**Fig 19****:** THz spectrum of 4-ClPh-PrDAP+C6Thy (1:1 complex).
**Fig 20****:** THz spectrum of 4-BrPh-PrDAP+C6Thy (1:1 complex).
**Fig 21****:** THz spectrum of Ph-DAP+C6Thy (1:1 complex) overlapped with PhDAP and C6-Thy spectra to demonstrate fine-tuning of resonances.

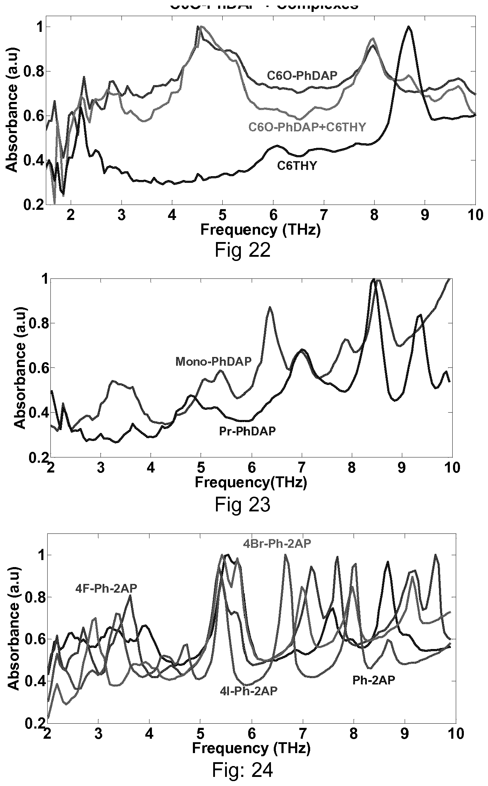

**Fig 22****:** THz spectrum of C6OPh-DAP+C6Thy (1:1 complex) overlapped with C6OPhDAP and C6-Thy spectra to demonstrate fine-tuning of resonances.
**Fig 23****:** THz spectra (overlapped) of monoPhDAP and Pr-PhDAP to demonstrate coarse tuning of Terahertz resonances by functional group substitution.
**Fig 24****:** THz spectra (overlapped) of Ph-2AP, 4-FPh-2AP, 4-BrPh-2AP and 4-IPh-2AP to demonstrate coarse tuning of Terahertz resonances by functional group substitution.

### DETAILED DESCRIPTION

The invention is defined by the appended claims. Any embodiment not falling under the scope of the appended claims does not form part of the invention. The present disclosure provides several novel compounds selected from a list of compounds (a) to (i), (aa), (cc) and binary molecular complexes based on these compounds.

Present disclosure provides a compound selected from a list of compounds (a) to (i), (aa), (cc) exhibiting resonances in the range of 0.1-10 THz. These molecules are herein referred to as THz molecular tags.

Use of a compound selected from a list of compounds (a) to (i), (aa), (cc) in authentication of a product by detecting the compound's resonance in the range of 0.1 to 10 THz ,
wherein the compounds are selected from:
a) N,N'-(pyridine-2,6diyl)dipropionamide (PrDAP);
b) N,N'-(pyridine-2,6-diyl)bis(4-(hexyloxy)benzamide) (C6OPhDAP);
c) N-(6-propionamidopyridin-2-yl)benzamide (Pr-PhDAP);
d) N-(6-benzamidopyridin-2-yl)-4-nitrobenzamide (4-NO₂Ph-PhDAP);
e) N-(6-(3-phenylureido)pyridin-2-yl)benzamide (PhU-PhAP);
f) 4-fluoro-N-(6-propionamidopyridin-2-yl)benzamide (4-FPh-PrDAP);
g) 4-chloro-N-(6-propionamidopyridin-2-yl)benzamide (4-ClPh-PrDAP);
h) 4-bromo-N-(6-propionamidopyridin-2-yl)benzamide (4-BrPh-PrDAP);
i) 4-iodo-N-(6-propionamidopyridin-2-yl)benzamide (4-IPh-PrDAP);
aa) 4-nitro-N-(6-propionamidopyridin-2-yl)benzamide (4-NO₂Ph-PrDAP);
cc) N,N'-(pyridine-2,6-diyl)bis(thiophene-2-carboxamide) (Th-DAP).

In another preferred embodiment of the present disclosure, the present disclosure provides a composition comprising a compound selected from a list of compounds (a) to (i), (aa) and (cc) and the substrate for authentication of a product by detecting the compound's resonance in the range of 0.1 to 10 THz.

In yet another embodiment of the present disclosure, the disclosure provides a composition comprising a compound selected from a list of compounds (a) to (i), (aa) and (cc) exhibiting resonances in the range of 0.1-10 THz which can be applied on various substrates (metals and/or plastics etc.) for absorbing Terahertz Radar frequencies.

In yet another aspect of the present disclosure, the disclosure provides use of a composition comprising a compound selected from a list of compounds (a) to (i), (aa) and (cc) and a substrate by tailoring refractive index at Terahertz frequencies. Using these compounds, refractive index can also be tuned at Terahertz frequencies.

In yet another embodiment of the present disclosure, the present disclosure provides a pathway for realizing molecules with optimized resonances in THz Radar frequencies, which can be used as absorber/coating materials on various metal/composite/plastic substrates.

In yet another embodiment of the present disclosure, the present disclosure provides a method for customization of resonances in Terahertz frequency region by tuning 1) molecular mass, 2) hydrogen bond strength, 3) inter/intra molecular bonds, 4) isomeric form, and 5) functional group. In yet another embodiment of the present disclosure, the disclosure provides novel compounds selected from a list of compounds (a) to (i), (aa) and (cc) for authentication of a product, wherein, the authentication of a product comprises detection of fake currency notes, security documents and counterfeit pharmaceutical drugs, brand protection, labeling of consumer and industrial products/solutions etc.

In still another embodiment of the present disclosure, the present disclosure provides a process of authenticating a product by embedding the product with THz tags, compounds selected from a list of compounds (a) to (i), (aa) and (cc), of the present disclosure, exhibiting resonance in the range of 0.1-10 THz, and detecting the Terahertz tag using a spectrometer/detector.

In an aspect the present disclosure, the present disclosure provides the solubility of novel compounds selected from a list of compounds (a) to (i), (aa) and (cc)in various solvents as shown below in Table: 1 demonstrating the easy incorporation of these THz molecular tags into various substrates:

**Table: 1**

| Sr No | Compound | dichloro-methane | Ethyl Acetate | Methanol | DMSO | DMF | THF | Acetone | Toluene | Diethyl Ether |
|---|---|---|---|---|---|---|---|---|---|---|
| 1 | Ph-DAP | S | S | S | S | S | S | S | I | I |
| 2 | Pr-DAP | S | S | S | S | S | S | S | S | S |
| 3 | C6-Thy | S | S | S | S | S | S | S | S | SΔ |
| 4 | 2-2'-DPA | S | S | S | S | S | S | S | S | S |
| 5 | PhU-Ph-AP | I | SΔ | I | S | S | S | S | Turbid | Turbid |
| 6 | 4FPh-PrDAP | S | S | S | S | S | S | S | PS | PS |
| 7 | 4-ClPh-PrDAP | S | S | S | S | S | S | S | PS | PS |
| 8 | 4-BrPh-PrDAP | S | S | S | S | S | S | S | PS | PS |
| 9 | 4-IPh-PrDAP | S | S | S | S | S | S | S | PS | S |
| 10 | 4NO₂Ph-PrDAP | S | S | S | S | S | S | S | I | PS |
| 11 | Th-DAP | S | S | S | S | S | S | S | I | I |
| 12 | Pr-PhDAP | S | S | S | S | S | S | S | I | PS |
| 13 | 4-NO₂-Ph-PhDAP | PS | S | S | S | S | S | S | I | I |
| 14 | C6O-PhDAP | S | S | S | S | S | S | S | S | S |
| 15 | PhDAP+C6 Thy (1:1 complex). | S | S | I | I | I | S | I | I | I |
| 16 | C6OPhDA P+C6Thy (1:1 complex) | S | S | I | I | I | S | I | S | I |
| 17 | PrDAP+ C6Thy (1:1 complex) | S | S | I | I | I | S | I | S | I |
| 18 | Pr-PhDAP +C6Thy (1:1 complex) | S | S | I | I | I | S | I | I | I |
| 19 | 4-FPh-PrDAP+C6 Thy (1:1 complex) | S | S | I | I | I | S | I | I | I |
| 20 | 4-ClPh-PrDAP+C6 Thy (1:1 complex). | S | S | I | I | I | S | I | I | I |
| 21 | 4-BrPh-PrDAP+C6 Thy (1:1 complex) | S | S | I | I | I | S | I | I | I |

| | | | | | | | | | | |
|---|---|---|---|---|---|---|---|---|---|---|
| **ABBREVIATIONS** S = Soluble; I = Insoluble; PS = Partially Soluble; SΔ = Soluble on heating at 50° C DMSO = dimethyl sulfoxide; DMF = N, N-dimethyl formamide; THF = tetrahydrofuran | | | | | | | | | | |

In yet another embodiment of the present disclosure, the disclosure provides use of a binary molecular complexe of:
a primary component selected from the list of compounds (a) to (i), (aa) and (cc), and
a secondary component selected from -hexyl-5-methylpyrimidine-2,4(1H, 3H)-dione (C6Thy) and Di-(pyridin-2-yl) amine (DPA),
in authentication of a product by detection of the complex's resonance in the range of 0.1 -10 THz . Accordingly, the disclosure encompasses binary molecular complexes selected from the group consisting of PhDAP+C6Thy (1:1 complex); C6OPhDAP+ C6Thy (1:1 complex); PrDAP+ C6Thy (1:1 complex); Pr-PhDAP+C6Thy (1:1 complex), 4-FPh-PrDAP+C6Thy (1:1 complex); 4-ClPh-PrDAP+C6Thy (1:1 complex); and 4-BrPh-PrDAP+C6Thy (1:1 complex). All these molecular complexes exhibit resonances in the range of 0.1 to 10 THz whose resonances can be fine-tuned easily.

In yet another embodiment of the present disclosure, the disclosure provides a process of authenticating a product which comprises embedding the product with Binary molecular complexes based on the novel compound selected from the list of compounds (a) to (i), (aa) and (cc), according to the disclosure exhibiting resonance in the range of 0.1-10 THz and detecting the Terahertz tag using a THz spectrometer/THz detector.

For the examples listed below, the prediction of resonances of a molecule in the THz region is carried out using Density Functional Theory (DFT) calculations.

### EXAMPLES

Examples not falling under the scope of the appended claims do not form part of the invention.

### Example 1

### N,N'-(pyridine-2,6-diyl)dibenzamide (PhDAP): (Fig: 1)

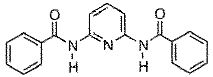

*Synthetic Procedure:* In a round bottom flask 2, 6-diaminopyridine (1g, 9.163 mmol) was dissolved in 40mL of dry dichloromethane and triethylamine (2.8mL, 27.72mmol) was added. Then, benzoyl chloride (2.5mL, 17.98 mmol) was added drop by drop and this mixture was stirred under inert atmosphere at room temperature for 15h. Reaction mixture was poured in water. Crude product was extracted in dichloromethane, organic layer was washed with water and dried over anhydrous sodium sulphate. The solvent was evaporated on rotary evaporator and crude product was purified by column chromatography on silica gel by eluting with ethyl acetate: hexane (40:60) mixture. The product was further purified by recrystallization using a methanol. Yield: 2.60 g (89%).
¹H NMR (200MHz, CDCl₃): *δ* ppm 7.54 (m, 6H), 7.90 (d, 4H, J=6Hz), 7.82 (t, 1H, J=8Hz), 8.10 (d, 2H, J=8Hz), 8.39 (s, 2H).

### Example 2

### N,N'-(pyridine-2,6 diyl)dipropionamide (PrDAP): (Fig: 2)

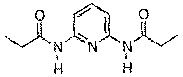

*Synthetic Procedure:* In a round bottom flask 2,6-diaminopyridine (1g, 9.163mmol) was dissolved with 50mL of dry dichloromethane and triethylamine (2.8ml, 27.72mmol) was added, and then propionyl chloride (1.76ml, 19.02mmol) was added drop by drop and this mixture was kept in inert atmosphere with stirring at room temp for 15h. After that reaction mixture was extracted with dichloromethane and washed with water. The organic layer was dried over anhydrous sodium sulphate. Product was purified by column chromatography on Silica gel, by eluting with ethyl acetate: hexane (40:60) mixture. The solvent was evaporated by the rotary evaporator under the reduced pressure and pure product dried in high vacuum. The product was further purified by recrystallization using a mixture of ethyl acetate and hexane (40%).Yield: 1.3gm, (64.4%).
NMR DATA: ¹H NMR (200MHz, CDCl₃): *δ* ppm 1.24 (t, 6H J=8Hz), 2.39 (q, 4H, J=12Hz), 1H (s, 7.57), 2H (d, 7.73 J=8Hz), 2H (d, 7.87, J=8Hz)

### Example 3

### N,N'-(pyridine-2,6-diyl)bis(4-(hexyloxy)benzamide) (C60PhDAP): (Fig: 3)

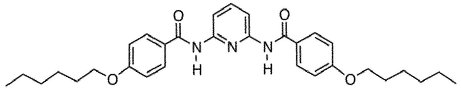

### Step 1

Synthetic Procedure: In the round bottom flask 4-hydroxy methyl benzoate (2g, 13.15mmol) was dissolved in 40ml of DMF and 1-Bromohexane (2.19ml, 13.26mmol) was added. Then potassium carbonate (5.4g, 39.07mmol) was added, and refluxed the reaction mixture at 70-80°C for 15h. Then the reaction mixture was poured in water and extracted with ethyl acetate. Organic layer was dried over anhydrous sodium sulphate. The product 4-hexyloxy methyl benzoate was purified by column chromatography on silica gel by eluting with 5% ethyl acetate in pet ether and the solvent was evaporated on the rotary evaporator under the reduced pressure and dried in high vacuum. Yield: 2.7g, (87.1 %).
¹H NMR (200MHz, CDCl₃):δ ppm, 0.81 (t, 3H), 1.26 (m, 6H), 1.68 (m, 2H, J=6Hz), 3.87 (m, 5H, J=6Hz), 6.81 (d, 2H, J=8Hz), 7.85 (d, 2H, J=8Hz).

### Step 2

Synthetic Procedure: In the round bottom flask 4-hexyloxy methyl benzoate (2.635g, 11.158mmol) dissolved in 25ml of THF and KOH (3.13g, 55.93mmol) dissolved in water (5ml, 0.278mmol) was added, and reflux the reaction mixture at 60°C for 15h. After the completion of the reaction, neutralised with dil. HCl and the precipitation was filtered and washed with water by extracting with ethyl acetate. The organic layer was dried over anhydrous sodium sulphate. The product 4-hexyloxy benzoic acid was purified by the column chromatography on silica gel by eluting with 20% of ethyl acetate in hexane. The solvent was evaporated on the rotary evaporator under the reduced pressure and dried in high vacuum. Yield: 1.7g, (68.8%).
¹H NMR (200MHz, CDCl₃):δ ppm, 0.91 (t, 3H), 1.37 (m, 6H), 1.81 (t, 2H), 4.02 (t, 3H, J=8Hz), 6.95 (d, 2H, J=8Hz), 8.07 (d, 2H, J=8Hz).

### Step 3

Synthetic Procedure: In round bottom flask 2,6-diaminopyridine (0.340g, 3.11mmol) and 4-hexyloxy benzoic acid (1.65g, 7.42mmol) was dissolved in 40mL of dichloromethane. Then 4-dimethylaminopyridine (0.348g, 2.85mmol) and EDCI-HCl (0.896g, 4.67mmol) were added and stirred under inert atmosphere at room temperature for 24h. After that reaction mixture was extracted with dichloromethane and the organic layer was washed with water, dried over anhydrous sodium sulphate. The product N,N'-(pyridine-2,6-diyl)bis(4- (hexyloxy)benzamide), was purified by column chromatography on silica gel by eluting with 15% of ethyl acetate in hexane. The solvent was evaporated by rotary evaporator under reduced pressure and dried under high vacuum. Yield: 0.413 g (25.7%).
¹H NMR (200MHz, CDCl₃):δppm 0.85 (t, 6H, J=6H), 1.27 (m, 12H, J=6H), 1.75 (q, 4H, J=6H), 3.95 (t, 4H, J=8H), 6.93 (d, 4H, J=10H), 7.81 (m, 7H, J=8H), 8.22 (s, 2H).

### Example 4

### 1-hexyl-5-methylpyrimidine-2,4(1H,3H)-dione (C6THY): (Fig: 4)

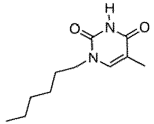

*Synthetic Procedure:* In a round bottom flask, thymine (8g, 63.43mmol) was dissolved in 60mL of dimethyl sulfoxide and 1-bromohexane (2.94mL, 17.81mmol) and K₂CO₃ (11.38g, 82.33mmol) were added. This mixture was stirred at 70-80°C for 15h. The suspension was filtered through sintered funnel, filter cake washed with dichloromethane and filtrate concentrated. The residue obtained was extracted in dichloromethane, washed with water, the organic layer dried over anhydrous sodium sulphate and solvent was evaporated by rotary evaporator under reduced pressure. The crude product was purified by column chromatography on silica with 25% of ethyl acetate in hexane and further purified by recrystallization using a mixture of dichloromethane:hexane (30:70). Yield: 6.14g (38.4%).
NMR DATA: ¹H NMR (200MHz, CDCl₃): *δ* ppm 3H (t, 0.88), 6H (m, 1.31), 3H (t, 1.70, J=4Hz), 3H(s, 1.92, J=6H), 2H (t, 3.68, J=12H), 1H(s, 6.97), 1H (s, 8.70).

### Example 5

### N-(6-propionamidopyridin-2-yl)benzamide (Pr-PhDAP): (Fig: 5)

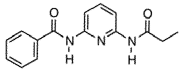

*Synthetic Procedure:* In a round bottom flask mono-PrDAP (1g, 6.06mmol) was dissolved in 40mL of dry dichloromethane and triethylamine (1.53mL, 15.14mmol) was added. Then benzoyl chloride (1.25ml, 8.99mmol) was added dropwise and this mixture was kept in inert atmosphere with stirring at room temperature for 15h. Then reaction mixture was extracted with dichloromethane and washed with water. The organic layer was dried over anhydrous sodium sulphate and solvent was evaporated on the rotary evaporator under reduced pressure. Product was purified by column chromatography on silica gel by eluting with 40% ethyl acetate in hexane. The product was further purified by recrystallization using a mixture of ethyl acetate and hexane (40:60). Yield: 1.5g, (68.2%).
¹H NMR (200 MHz, CDCl₃) *δ* ppm 1.25 (t, 3H, J=8Hz), 2.41 (q, 2H, J=8Hz), 7.53 (m, 3H), 7.66 (s, 1H), 7.76 (t, 1H, J=8Hz), 7.88 (m, 2H), 8.05 (d, 1H, J=8Hz), 8.30 (s, 1H).

### Example 6

### N-(6-benzamidopyridin-2-yl)-4-nitrobenzamide (4-NO₂Ph-PhDAP): (Fig: 6)

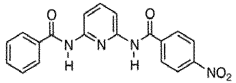

*Synthetic Procedure:* In a round bottom flask mono-PhDAP (1g, 4.69mmol) and 4-nitrobenzoic acid (1.18g, 7.06mmol) were dissolved in 40mL of N,N-dimethyl formamide and 4-dimethylaminopyridine (0.57g, 4.66mmol) and EDCI-HCl (1.82g, 11.72mmol) were added under inert atmosphere at room temperature for 24h. Then reaction mixture was extracted with dichloromethane and washed with water the organic layer was dried over anhydrous sodium sulphate and solvent was evaporated on rotary evaporator under reduced pressure. The product was purified by column chromatography on silica gel by eluting with 50% of ethyl acetate in hexane and dried under high vacuum. Yield: 1.12g (66%).
¹H NMR (200 MHz, CDCl₃) *δ* ppm: 7.57 (m, 3H), 7.84-7.94 (m, 3H), 8.12-8.21 (m, 4H), 8.36 (m, 4H).

### Example 7

### N-(6-(3-phenylureido)pyridin-2-yl)benzamide (PhU-PhAP): (Fig: 7)

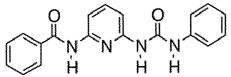

*Synthetic Procedure:* Mono-PhDAP (200mg, 0.938mmol) and phenyl isocyanate (0.11mL, 0.923mmol) were dissolved in 60 mL of dry dichloromethane. The solution was stirred under reflux in inert atmosphere for 24 h. After this time the solution product precipitated. Reaction mixture was filtered using Whatmann filter paper, residue was washed with dichloromethane and dried under high vacuum. Yield 0.212 g (68%).
¹H NMR (200MHz, CDCl₃): *δ* ppm: 7.03 (m, 2H), 7.34 (t, 2H, J=8Hz), 7.62 (m, 7H), 7.98 (dd, 2H, J=8Hz), 9.55 (s, 1H), 10.78, 10.84 (d, 2H).

### Example 8

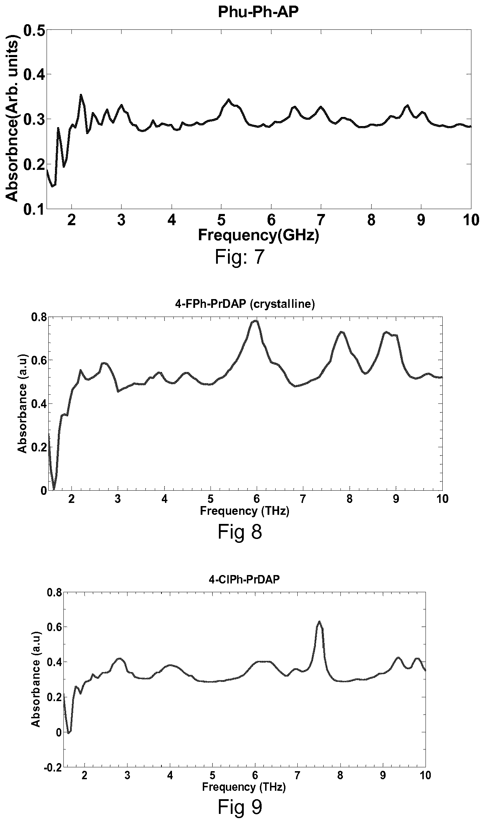

### 4-fluoro-N-(6-propionamidopyridin-2-yl)benzamide (4-FPh-PrDAP): (Fig 8)

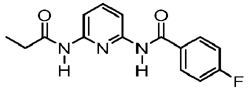

In the round bottom flask mono Pr-DAP (0.3g, 1.81mmol) was dissolved in 40ml of dry DCM and triethylamine (0.31mL, 2.18mmol) was added. Then 4-fluorobenzoyl chloride (0.26 mL, 2.18mmol) was added drop by drop. This mixture was kept in inert atmosphere with stirring at room temp for 15h. After that, reaction mixture was washed with water by extracting with DCM. The organic layer was dried over anhydrous sodium sulphate. Product purified by column chromatography on silica gel by eluting with 30% ethyl acetate and hexane. The solvent was evaporated by the rotary evaporator under reduced pressure and pure product was dried under high vacuum. Yield: 0.49g (94%).
¹H NMR (400MHz, CDCl₃): *δ* ppm 1.26 (t, 3H, J=8Hz), 2.43 (q, 2H, J=8Hz), 7.19 (t,2H,J=8Hz), 7.72 (br,s, 1H), 7.78 (t,1H,J=8Hz), 7.93 (m, 3H), 8.03 (t,1H,J=8Hz), 8.27 (br,s,1H).

### Example 9

### 4-chloro-N-(6-propionamidopyridin-2-yl)benzamide (4-ClPh-PrDAP): (Fig 9)

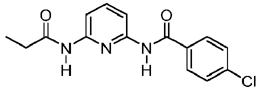

In the round bottom flask mono Pr-DAP (0.2g, 1.21mmol) was dissolved in 40ml of dry dichloromethane and triethylamine (0.20mL, 1.45mmol) was added. Then 4-chlorobenzoyl chloride (0.187 mL, 1.45mmol) was added drop by drop. This mixture was kept in inert atmosphere with stirring at room temp for 15h. Then it was extracted with DCM and washed with water. The organic layer was dried over anhydrous sodium sulphate. Product was purified by column chromatography on silica gel by eluting with 30% ethyl acetate in hexane. The solvent was evaporated under reduced pressure and dried under high vacuum. Yield: 0.21g (56%).
¹H NMR (400MHz, CDCl₃): δ ppm 1.27 (t, 3H, J=8Hz), 2.44 (q, 2H, J=8Hz), 7.50 (d, 2H, J=8Hz), 7.72 (s, 1H), 7.78 (t, 1H, J=8Hz), 7.84 (d, 2H, J=8Hz), 7.97 (d, 1H, J=8Hz), 8.04 (d, 1H, J=8Hz), 8.26 (s, 1H).

### Example 10

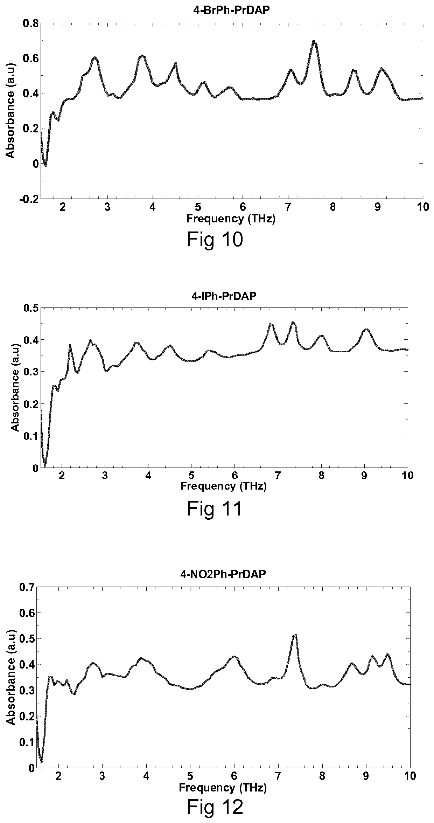

### 4-bromo-N-(6-propionamidopyridin-2-yl)benzamide (4-BrPh-PrDAP): (Fig 10)

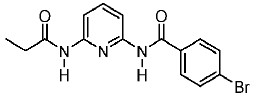

In the round bottom flask mono Pr-DAP (0.2g, 1.21mmol) was dissolved in 40ml of dry dichloromethane and triethylamine (0.20mL, 1.45mmol) was added. Then 4-bromobenzoyl chloride (0.32 gm, 1.45mmol) in dry DCM was added drop by drop. This mixture was kept in inert atmosphere with stirring at room temp for 15h. After that, reaction mixture was washed with water by extracting with DCM. The organic layer was dried over anhydrous sodium sulphate. Product purified by column chromatography on silica gel by eluting with 30% ethyl acetate and hexane. The solvent was evaporated by the rotary evaporator under reduced pressure and pure product was dried under high vacuum. Yield: 0.24 g (57%).
¹H NMR (200MHz, CDCl₃): δ ppm 1.27 (t, 3H, J=8Hz), 2.42 (q, 2H, J=8Hz), 7.59 (d, 2H, J=8Hz), 7.67 (m, 2H), 7.75,7.79 (m, 3H), 7.95 (d, 2H, J=8Hz), 8.02 (d, 1H, J=8Hz), 8.20 (s, 1H).

### Example 11

### 4-iodo-N-(6-propionamidopyridin-2-yl)benzamide (4-IPh-PrDAP): (Fig 11)

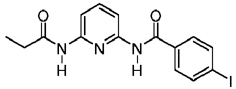

In the round bottom flask mono Pr-DAP (0.2g, 1.21mmol) was dissolved in 40 ml of dry dichloromethane and triethylamine (0.25mL, 1.81mmol) was added. Then 4-iodobenzoyl chloride (0.48 gm, 1.81mmol) in dry DCM was added drop by drop. This mixture was kept in inert atmosphere with stirring at room temp for 15h. After that, reaction mixture was washed with water after extracting with DCM. The organic layer was dried over anhydrous sodium sulphate. Product purified by column chromatography on silica gel by eluting with 30% ethyl acetate and hexane. The solvent was evaporated by the rotary evaporator under reduced pressure and pure product was dried under high vacuum. Yield: 0.26 g (55%).
¹H NMR (200MHz, CDCl₃): δ ppm 1.27 (t, 3H, J=8Hz), 2.43 (q, 2H, J=8Hz), 7.59 (d, 2H, J=8Hz), 7.60 (d, 2H,J=8Hz), 7.77,(t, 1H, J=8Hz), 7.85 (d, 2H, J=8Hz), 7.95 (d, 1H, J=10Hz), 8.02 (d, 1H, J=10Hz), 8.20(s,1H).

### Example 12

### 4-nitro-N-(6-propionamidopyridin-2-yl)benzamide(4-NO₂Ph-PrDAP): (Fig 12)

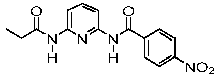

Mono Pr-DAP (0.1g, 0.6mmol) was added in ethyl acetate 25mL, purified by washing with 5% sodium carbonate solution, saturated sodium chloride solution, drying over anhydrous magnesium sulphate. Mixture of mono-Pr-DAP and purified ethyl acetate was cooled to 5° C. Then triethyl amine (0.1mL, 0.72mmol) was added. Then 4-nitrobenzoyl chloride (0.14g, 0.72mmol) dissolved in purified ethyl acetate was added drop wise at such a rate to maintain the temperature below 10° C. The ice bath was removed upon complete addition of 4-nitro benzoyl chloride solution and the reaction stirred for 24 hours. The reaction mixture was then filtered on a by Whitman filter paper. The filtrate washed three times with 5%NaOH solution and once with water, to remove unreacted 4-Nitrobenzoyl chloride, and washed once with saturated sodium chloride, dried over anhydrous magnesium sulphate, filtered through sodium sulphate funnel. The solvent was evaporated by the rotary evaporator under reduced pressure and pure product was dried under high vacuum. Yield: 0.11g (58%).
¹H NMR (400MHz, CDCl₃): *δ* ppm 1.27 (t, 3H, J = 8Hz), 2.43 (q, 2H, J = 8Hz), 7.60 (brs, 1H), 7.80 (t, 1H, J=8Hz), 8.10 (m,4H), 8.35 (m, 3H).

### Example 13

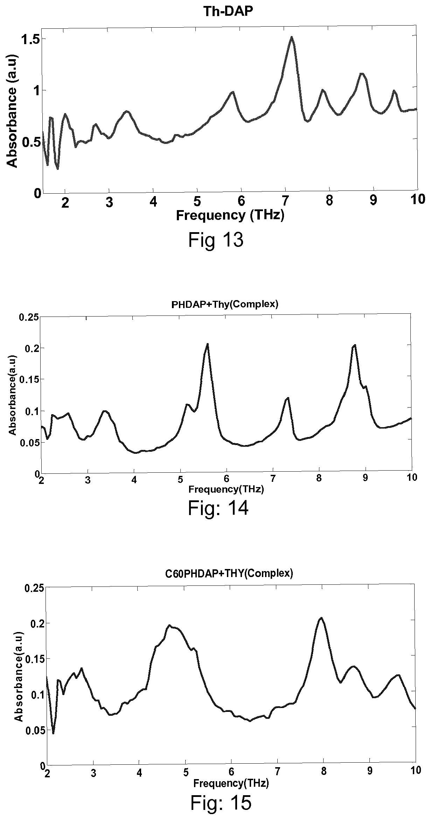

### N,N'-(pyridine-2,6-diyl)bis(thiophene-2-carboxamide) (Th-DAP): (Fig 13)

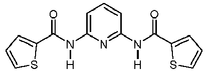

In the round bottom flask thiophene-2-carboxylic acid (1g, 7.8mmol) was dissolved in 30 mL of dry dichloromethane and thionyl chloride (0.74mL, 10.1mmol) was added slowly by dropping funnel followed by 1-2 drops of dry DMF. This mixture was kept in inert atmosphere with stirring at room temp for 24 h. After that, all the solvent was removed by vacuum distillation without heating the reaction mixture. In two necked round bottom flask 2,6-DAP (0.2g, 1.83mmol) was dissolved in dry dichloromethane, and dry pyridine (0.32mL, 4.03mmol) was added. This reaction mixture was stirred for 15 min. Previously made thiophene-2-carbonyl chloride (0.43g, 4.03mmol) was added drop by drop to the amine containing reaction mixture and stirred for 24 h. Completion of reaction was confirmed by TLC, reaction mixture was washed with water and extracted in dichloromethane. The organic layer was dried over anhydrous sodium sulfate. Product was purified by column chromatography on silica gel by eluting with 30% ethyl acetate in hexane. The solvent was evaporated under reduced pressure and dried under high vacuum. Yield: = 0.51g (85%).
¹H NMR (400MHz, CDCl₃): δ ppm 7.14 (t, 2H, J=4Hz), 7.58, 7.65 (dd, 4H), 7.77 (t, 1H, J=8Hz), 8.01 (d, 2H, J=8Hz), 8.19 (s, 2H).

### Example 14

### Hydrogen bonded complexes of heterocomplementary pairs (DAP and C6THY):

### General Procedure for preparation of complexes:

Equimolar mixture of N,N'-diacylamino pyridine (DAP derivative) and C6THY was prepared in minimum amount of chloroform by mixing solutions of the two compounds prepared in chloroform separately. The solution mixture was stirred at room temperature for 10 min to allow for complex formation. Solvent was evaporated on rotary evaporator under vacuum at room temperature and dried under high vacuum. The solid obtained was used for spectroscopic studies.
1. PhDAP+C6Thy (1:1 complex) : (Fig: 14) 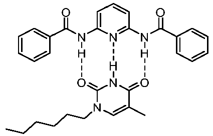
2. C6OPhDAP+ C6Thy (1:1 complex): (Fig: 15) 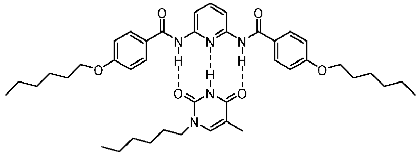
3. PrDAP+ C6Thy (1:1 complex): (Fig: 16) 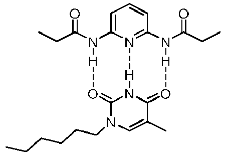
4. Pr-PhDAP+C6Thy (1:1 complex): (Fig: 17)

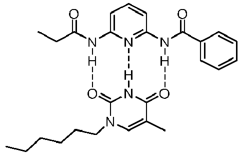

### Example: 15

### Fine tuning of THz resonances by using hydrogen bonded complexes

1. THz spectrum of Ph-DAP+C6Thy (1:1 complex) overlapped with PhDAP and C6-Thy spectra. (Fig: 21)
2. THz spectrum of C6OPh-DAP+C6Thy (1:1 complex) overlapped with C6OPhDAP and C6-Thy spectra. (Fig: 22)

### Example 16

### Coarse Tuning of Terahertz resonances by functional group substitution

1. THz spectra (overlapped) of monoPhDAP and Pr-PhDAP to demonstrate coarse tuning of Terahertz resonances by functional group substitution (Fig: 23).
2. THz spectra (overlapped) of Ph-2AP, 4-FPh-2AP, 4-BrPh-2AP and 4-IPh-2AP to demonstrate coarse tuning of Terahertz resonances by functional group substitution (Fig: 24).**ADVANTAGES OF**

### THE INVENTION

a. Tagging of products is easier by using the novel substituted heterocyclic and/or aromatic compounds containing amide and/or urea groups.
*b*. Customizable resonances in the range of 0.1 to 10 THz.

## Claims

1. Use of a compound selected from a list of compounds (a) to (i), (aa) and (cc) in authentication of a product by detecting the compound's resonance in the range of 0.1 -10 THz,
wherein the compounds are selected from:
a. N,N'-(pyridine-2,6diyl)dipropionamide (PrDAP);
b. N,N'-(pyridine-2,6-diyl)bis(4-(hexyloxy)benzamide) (C6OPhDAP);
c. N-(6-propionamidopyridin-2-yl)benzamide (Pr-PhDAP);
d. N-(6-benzamidopyridin-2-yl)-4-nitrobenzamide (4-NO₂Ph-PhDAP);
e. N-(6-(3-phenylureido) pyridin-2-yl)benzamide (PhU-PhAP);
f. 4-fluoro-N-(6-propionamidopyridin-2-yl)benzamide (4-FPh-PrDAP);
g. 4-chloro-N-(6-propionamidopyridin-2-yl)benzamide (4-ClPh-PrDAP);
h. 4-bromo-N-(6-propionamidopyridin-2-yl)benzamide (4-BrPh-PrDAP);
i. 4-iodo-N-(6-propionamidopyridin-2-yl)benzamide (4-IPh-PrDAP);
aa. 4-nitro-N-(6-propionamidopyridin-2-yl)benzamide (4-NO₂Ph-PrDAP);
cc. N,N'-(pyridine-2,6-diyl)bis(thiophene-2-carboxamide) (Th-DAP).

2. Use of a binary molecular complex of:
a primary component selected from the list of compounds (a) to (i), (aa) and (cc) of claim 1, and
a secondary component selected from 1-hexyl-5-methylpyrimidine-2,4(1H, 3H)-dione (C6Thy) and Di-(pyridin-2-yl) amine (DPA),
in authentication of a product by detection of the complex's resonance in the range of 0.1 -10 THz.

3. Binary molecular complexes according to claim 2, comprising:
a) PhDAP+C6Thy (1:1 complex);
b) C6OPhDAP+C6Thy (1:1 complex);
c) PrDAP+C6Thy (1:1 complex);
d) Pr-PhDAP+C6Thy (1:1 complex);
e) 4-FPh-PrDAP+C6Thy (1:1 complex);
f) 4-ClPh-PrDAP+C6Thy (1:1 complex); and
g) 4-BrPh-PrDAP+C6Thy (1:1 complex).

4. Use according to claim 1, wherein the authentication of a product comprises detection of fake currency notes, security documents and counterfeit pharmaceutical drugs, brand protection, labeling of consumer and industrial products/solutions .

5. A process for authentication of a product comprising embedding the product with a compound according to claim 1 and/or a binary molecular complex according to claim 2, and detecting the tag's resonance in the range of 0.1 to 10 THz using a THz spectrometer/THz detector.

6. Use of a composition comprising a compound of claim 1 and a substrate for authentication of a product by detecting the compound's resonance in the range of 0.1-10 THz.

7. Use according to claim 6, wherein the substrate is selected from metals and/or, plastics for absorbing Terahertz Radar frequencies.

8. Use according to claim 6 or 7 by tailoring the refractive index at Terahertz frequencies.

## Patentansprüche

1. Verwendung einer Verbindung, die aus einer Liste von Verbindungen (a) bis (i), (aa) und (cc) bei Authentifizierung eines Produkts durch Erkennen der Resonanz der Verbindung in dem Bereich von 0,1-10 THz ausgewählt wird,
wobei die Verbindungen aus Folgendem ausgewählt sind:
a. N,N'-(Pyridin-2,6-diyl)dipropionamid (PrDAP);
b. N,N'-(Pyridin-2,6-diyl)bis(4-(hexyloxy)benzamid) (C60PhDAP ) ;
c. N-(6-Propionamidopyridin-2-yl)benzamid (Pr-PhDAP);
d. N-(6-Benzamidopyridin-2-yl)-4-nitrobenzamid (4-NO₂Ph-PhDAP);
e. N-(6-(3-Phenylureido)pyridin-2-yl)benzamid (PhU-PhAP);
f. 4-Fluor-N-(6-propionamidopyridin-2-yl)benzamid (4-FPh-PrDAP) ;
g. 4-Chlor-N-(6-propionamidopyridin-2-yl)benzamid (4-ClPh-PrDA P) ;
h. 4-Brom-N-(6-propionamidopyridin-2-yl)benzamid (4-BrPh-PrDAP ) ;
i. 4-Iod-N-(6-propionamidopyridin-2-yl)benzamid (4-IPh-PrDAP);
aa. 4-Nitro-N-(6-propionamidopyridin-2-yl)benzamid (4-NO₂Ph-PrD AP) ;
cc. N,N'-(Pyridin-2,6-diyl)bis(thiophen-2-carboxamid) (Th-DAP ).

2. Verwendung eines binären Molekularkomplexes, der aus Folgendem besteht:
einer primären Komponente, die aus der Liste von Verbindungen (a) bis (i), (aa) und (cc) nach Anspruch 1 ausgewählt ist, und einer sekundären Komponente, die aus 1-Hexyl-5-methylprimidin-2,4(1H, 3H)-dion (C6Thy) und Di-(pyridin-2-yl)amin (DPA) ausgewählt ist,
bei Authentifizierung eines Produkts durch Erkennen der Resonanz des Komplexes in dem Bereich von 0,1-10 THz.

3. Binärer Molekularkomplex nach Anspruch 2, Folgendes umfassend:
a) PhDAP+C6Thy (1:1 Komplex);
b) C6OPhDAP+C6Thy (1:1 Komplex);
c) PrDAP+C6Thy (1:1 Komplex);
d) Pr-PhDAP+C6Thy (1:1 Komplex);
e) 4-FPh-PrDAP+C6Thy (1:1 Komplex);
f) 4-ClPh-PrDAP+C6Thy (1:1 Komplex); und
g) 4-BrPh-PrDAP+C6Thy (1:1 Komplex).

4. Verwendung nach Anspruch 1, wobei die Authentifizierung eines Produkts das Erkennen von gefälschten Banknoten und Sicherheitsdokumenten sowie von nachgebildeten pharmazeutischen Wirkstoffen, nachgebildetem Markenschutz und das Labeln von Verbraucher- und Industrieprodukten und -lösungen umfasst.

5. Verfahren zur Authentifizierung eines Produkts, welches das Einbetten einer Verbindung nach Anspruch 1 und/oder eines binärem Molekularkomplexes nach Anspruch 2 in das Produkt und das Erkennen der Resonanz der Markierung in dem Bereich von 0,1 bis 10 THz unter Verwendung eines Terahertzspektrometers/ Terahertzdetektors umfasst.

6. Verwendung einer Zusammensetzung, die eine Verbindung nach Anspruch 1 und ein Substrat zur Authentifizierung eines Produkts durch Erkennen der Resonanz der Verbindung in dem Bereich von 0,1-10 THz umfasst.

7. Verwendung nach Anspruch 6, wobei das Substrat aus Metallen und/oder Kunststoffen zum Absorbieren von Terahertz-Radarfrequenzen ausgewählt ist.

8. Verwendung nach Anspruch 6 oder 7 durch Anpassen des Brechungsindexes bei Terahertz-Frequenzen.

## Revendications

1. Utilisation d'un composé sélectionné dans une liste de composés (a) à (i), (aa) et (cc) dans l'authentification d'un produit par détection de la résonance du composé dans la plage de 0,1 à 10 THz,
dans laquelle les composés sont sélectionnés parmi :
a. N,N'-(pyridine-2,6-diyl)dipropionamide (PrDAP) ;
b. N,N'-(pyridine-2,6-diyl)bis(4-(hexyloxy)benzamide) (C6OPhDAP) ;
c. N-(6-propionamidopyridin-2-yl)benzamide (Pr-PhDAP) ;
d. N-(6-benzamidopyridin-2-yl)-4-nitrobenzamide (4-NO₂Ph-PhDAP) ;
e. N-(6-(3-phényluréido) pyridin-2-yl) benzamide (PhU-PhAP) ;
f. 4-fluoro-N-(6-propionamidopyridin-2-yl)benzamide (4-FPh-PrDAP) ;
g. 4-chloro-N-(6-propionamidopyridin-2-yl)benzamide (4-ClPh-PrDAP) ;
h. 4-bromo-N-(6-propionamidopyridin-2-yl)benzamide (4-BrPh-PrDAP) ;
i. 4-iodo-N-(6-propionamidopyridin-2-yl)benzamide (4-IPh-PrDAP) ;
aa. 4-nitro-N-(6-propionamidopyridin-2-yl)benzamide (4-NO₂Ph-PrDAP) ;
cc. N,N'-(pyridine-2,6-diyl)bis(thiophène-2-carboxamide) (Th-DAP) .

2. Utilisation d'un complexe moléculaire binaire de :
un composant primaire sélectionné dans la liste de composés (a) à (i), (aa) et (cc) de la revendication 1, et
un composant secondaire sélectionné parmi la 1-hexyl-5-méthylpyrimidine-2,4(1H,3H)-dione (C6Thy) et la Di-(pyridin-2-yl) amine (DPA),
dans l'authentification d'un produit par détection de la résonance du complexe dans la plage de 0,1 à 10 THz.

3. Complexes moléculaires binaires selon la revendication 2, comprenant :
a) PhDAP + C6Thy (complexe 1/1) ;
b) C6OPhDAP + C6Thy (complexe 1/1) ;
c) PrDAP + C6Thy (complexe 1/1) ;
d) Pr-PhDAP + C6Thy (complexe 1/1) ;
e) 4-FPh-PrDAP + C6Thy (complexe 1/1) ;
f) 4-ClPh-PrDAP + C6Thy (complexe 1/1) ; et
g) 4-BrPh-PrDAP + C6Thy (complexe 1/1).

4. Utilisation selon la revendication 1, dans laquelle l'authentification d'un produit comprend la détection de faux billets, documents de sécurité et médicaments contrefaits, la protection de marques, le marquage de produits/solutions de consommation et industriels.

5. Procédé d'authentification d'un produit comprenant l'intégration dans le produit d'un composé selon la revendication 1 et/ou d'un complexe moléculaire binaire selon la revendication 2, et la détection de la résonance du marqueur dans la plage de 0,1 à 10 THz en utilisant un spectromètre THz/détecteur THz.

6. Utilisation d'une composition comprenant un composé selon la revendication 1 et un substrat pour l'authentification d'un produit par détection de la résonance du composé dans la plage de 0,1 à 10 THz.

7. Utilisation selon la revendication 6, dans laquelle le substrat est sélectionné parmi les métaux et/ou les plastiques pour l'absorption des fréquences radar térahertz.

8. Utilisation selon la revendication 6 ou 7 par personnalisation de l'indice de réfraction à des fréquences térahertz.
